Europäisches Patentamt

European Patent Office

Office européen des brevets

(11) Veröffentlichungsnummer: **0 517 274 A1**

# EUROPÄISCHE PATENTANMELDUNG

(21) Anmeldenummer: **92109602.0**

(51) Int. Cl.5: **A61K 9/00**, A61K 31/60

(22) Anmeldetag: **05.06.92**

(30) Priorität: **07.06.91 CH 1690/91**
**05.07.91 DE 4122337**

(43) Veröffentlichungstag der Anmeldung:
**09.12.92 Patentblatt 92/50**

(84) Benannte Vertragsstaaten:
**PT**

(71) Anmelder: **CEDONA PHARMACEUTICALS B.V.**
**P.O. Box 850, Oudeweg 147**
**NL-2003 RW Haarlem(NL)**

(72) Erfinder: **Wittebrood, Adrianus J.**
**Vederkruid 8**
**NL-1991 HB Velserbroek(NL)**
Erfinder: **De Jong, Adrianus P.**
**Valeriuslaan 4**
**NL-1985 EW Driehuis(NL)**
Erfinder: **Bron, Jan**
**Slingelandseweg 1**
**NL-3381 KZ Giessenburg(NL)**

(74) Vertreter: **Rupp, Herbert, Dr. et al**
**Byk Gulden Lomberg Chemische Fabrik**
**GmbH, Byk-Gulden-Strasse 2, Postfach 10 03**
**10**
**W-7750 Konstanz(DE)**

(54) **Pharmazeutische Klistierzubereitung.**

(57) Es werden 5-Aminosalicylsäure enthaltende Suspensionen für Klistieranwendungen beschrieben, die sich durch einen Gehalt an Titandioxid auszeichnen. Die neuen Suspensionen zeigen neben erhöhter Licht- und Oxidationsstabilität eine verbesserte Resuspendierbarkeit.

EP 0 517 274 A1

## Technisches Gebiet

Die Erfindung betrifft eine Klistierzubereitung für 5-Aminosalicylsäure.

## Stand der Technik

5-Aminosalicylsäure (5-ASA) ist ein bekannter Wirkstoff, der vor allem zur Behandlung entzündlicher Darmerkrankungen, wie Colitis ulcerosa, eingesetzt wird. Besonders günstig ist die Anwendung von 5-ASA als Klistierzubereitung, da hiermit das wirksame Prinzip direkt an den Ort der krankhaften Veränderungen gebracht werden kann. Bei diesen Zubereitungen bereitet allerdings die bekannte chemische Instabilität von 5-ASA in Lösungen oder Suspensionen erhebliche Schwierigkeiten. In der Vergangenheit wurden verschiedene Wege beschritten, um vor allem die Licht- und Sauerstoffempfindlichkeit von 5-ASA in den Griff zu bekommen.

So wird in der US-PS 4657900 eine Klistierzubereitung vorgeschlagen, bei der hochreine 5-ASA als wäßrige Suspension vorliegt, die nach Herstellung unter Sauerstoffausschluß und Zusatz von Bisulfit als Antioxidans in einem opaken Polyethylen-Rektalapplikator versiegelt wird, der seinerseits in einem Polyester/Aluminiumfolie/Polyethylen-Beutel hitzeversiegelt wird.

Aus der US-PS 4664256 ist eine sehr ähnliche Verabreichungsform bekannt, in der außer Bisulfit als Antioxidans noch ein Chelatbildner, wie z.B. Ethylendiamintetraacetat (EDTA) enthalten ist.

Das Problem der Instabilität bei Klistierzubereitungen wird durch diese Maßnahmen anscheinend leidlich gelöst.

Abgesehen von der aufwendigen Verpackung bei den 5-ASA-Klistierzubereitungen nach dem Stand der Technik zeigen die Zubereitungen jedoch Probleme was die Resuspendierbarkeit der 5-ASA-Suspension vor dem Gebrauch angeht. Die bekannten Zubereitungen müssen nämlich vor Gebrauch sehr kräftig und ausdauernd geschüttelt werden, um eine für die Anwendung wünschenswerte homogene Verteilung des Wirkstoffs zu erreichen. Es versteht sich von selbst, daß diese mühsame Resuspendierung insbesondere von gebrechlichen Patienten nur unzureichend bewältigt wird und insgesamt für die Patienten-Compliance nicht förderlich ist.

## Beschreibung der Erfindung

Es wurde nun überraschenderweise gefunden, daß sich 5-ASA-Klistierzubereitungennach dem Stand der Technik durch den Zusatz von Titandioxid in mehrfacher Hinsicht verbessern lassen. Die Licht und Oxidationsstabilität von 5-ASA-Suspensionen erhöht sich durch diesen erfindungssgemäßen Zusatz derart, daß es nicht mehr notwendig ist, die 5-ASA-Suspension in einem doppelwandigen Behälter zur Verfügung zu stellen. Als weiterer Vorteil ergibt sich, daß die Stabilität der Suspension so erhöht wird, daß das lästige Resuspendieren vor der Anwendung durch Schütteln entfallen kann.

Gegenstand der Erfindung ist daher eine wäßrige 5-Aminosalicylsäure-Klistiersuspensionszubereitung, die dadurch gekennzeichnet ist, daß neben den üblichen Hilfsstoffen Titandioxid enthalten ist.

Unter üblichen Hilfsstoffen werden solche Hilfsstoffe verstanden, die für Suspensionen üblicherweise eingesetzt werden und solche, die zur Stabilisierung von 5-ASA-Suspensionen gebräuchlich sind. Zur ersten Gruppe zählen z.B. die üblichen viskositätserhöhenden Stoffe, Konservierungsmittel, wie z.B. Benzoesäure, den pH-Wert regulierende Stoffe, wie Puffer, Chelatbildner, wie z.B. EDTA, und Antioxidantien, wie insbesondere die bei 5-ASA-Zubereitungen üblichen Bisulfite.

Titandioxid wird erfindungsgemäß in einer Menge von 0,1 bis 3, vorzugsweise 0,1 bis 1 und insbesondere 0,5 Gew.-% zugefügt.

5-ASA ist in den erfindungsgemäßen Suspensionen in einer Menge von 0,5 bis 10, vorzugsweise 2 bis 6 und insbesondere etwa 4 Gew.-% enthalten.

Bisulfite als Antioxidantien sind in den erfindungsgemäßen Suspensionen in einer Menge von 0,05 bis 0,5, vorzugsweise 0,1 bis 0,2 und insbesondere etwa 0,15 Gew.-% enthalten.

Viskositätserhöhende Stoffe sind in den erfindungsgemäßen Suspensionen in einer Menge von 0,05 bis 2, vorzugsweise 0,1 bis 1 und insbesondere etwa 0,5 Gew.-% enthalten.

Chelatbildner sind in der erfindungsgemäßen Suspension in einer Menge von 0,01 bis 0,5, vorzugsweise 0,05 bis 0,2 und insbesondere etwa 0,1 Gew.% enthalten.

Die Herstellung der erfindungsgemäßen Suspension erfolgt nach den aus dem Stand der Technik bekannten Verfahren unter Sauerstoffausschluß. Die fertige Suspension wird dann unter Sauerstoffausschluß in übliche flexible Klistierapplikatoren abgefüllt, die vorzugsweise aus opakem Kunststoff hergestellt werden.

Herstellungsbeispiel

Nach den üblichen Verfahren wird eine Suspension hergestellt und in Klistierbehälter abgefüllt, die pro 50 g Klistierpackung folgende Bestandteile enthält:

| | |
|---|---|
| 5-Aminosalicylsäure | 2,000 g |
| Natriumdihydrogenphosphat x 2$H_2$O | 0,500 g |
| Natriumedetat | 0,050 g |
| Benzoesäure | 0,100 g |
| Titandioxid E 171 | 0,250 g |
| Natriumdisulfit | 0,075 g |
| Xanthan Gummi | 0,250 g |
| Natriumhydroxid-Lösung 10 M | (bis pH 4,0) |
| Gereinigtes Wasser | bis 50,00 g |

**Patentansprüche**

1. Wäßrige 5-Aminosalicylsäure-Klistiersuspensionszubereitung, dadurch gekennzeichnet, daß neben üblichen Hilfsstoffen Titandioxid enthalten ist.

2. Zubereitung nach Anspruch 1, dadurch gekennzeichnet, daß 0,1 bis 3 Gew.-% Titandioxid enthalten sind.

3. Zubereitung nach Anspruch 1, dadurch gekennzeichnet, daß 5-Aminosalicylsäure in einer Menge von 0,5 bis 10 Gew.% enthalten ist.

4. Zubereitung nach Anspruch 1, dadurch gekennzeichnet, daß als Hilfsstoffe viskositätserhöhende Stoffe, Konservierungsstoffe, Chelatbildner, Puffer und Antioxidantien enthalten sind.

Europäisches
Patentamt

**EUROPÄISCHER RECHERCHENBERICHT**

Nummer der Anmeldung

EP    92 10 9602

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | FR-A-2 647 344 (PHYSIOPHARM)<br>* Seite 2, Zeile 11 - Seite 3, Zeile 2 *<br>--- | 1-4 | A61K9/00<br>A61K31/60 |
| Y | EP-A-0 395 329 (SMITH KLINE & FRENCH LABORATORIES LTD)<br>* Seite 2, Zeile 1 - Zeile 10 *<br>* Seite 3, Zeile 31 - Zeile 39 *<br>* Seite 4; Beispiel 1 *<br>--- | 1-4 | |
| A | EP-A-0 291 159 (DAK-LABORATORIET A/S)<br>* Seite 3, Zeile 47 - Zeile 50 *<br>* Seite 20; Beispiel 32 *<br>--- | 1-4 | |
| A | EP-A-0 398 207 (MARION MERREL DOW INC.)<br>* Seite 3; Beispiel 1 *<br>--- | 1-4 | |
| A,D | US-A-4 657 900 (POWELL D.R. ET AL)<br>* Seite 10 - Seite 11; Beispiel 3 *<br><br>----- | 1-4 | |

RECHERCHIERTE SACHGEBIETE (Int. Cl.5)

A61K

Der vorliegende Recherchenbericht wurde für alle Patentansprüche erstellt

| Recherchenort | Abschlußdatum der Recherche | Prüfer |
|---|---|---|
| DEN HAAG | 14 SEPTEMBER 1992 | BOULOIS O. |